# EUROPEAN PATENT APPLICATION

(11) **EP 4 257 962 A1**
(43) Date of publication of application: **11.10.2023**
(21) Application number: 21900960.2
(22) Date of filing: 30.11.2021
(51) Int. Cl.: G01N 27/12, G01N 33/00

(54) **GAS SPECIES IDENTIFICATION METHOD OF PHOTOACTIVE GAS SENSOR BY USING VARIABLE LIGHT RADIATION**

(30) Priority: 04.12.2020 KR 20200168210
(71) Applicant: Korea Advanced Institute of Science and Technology, Daejeon 34141 (KR)
(72) Inventor: PARK, In kyu, Daejeon 34141 (KR); CHO, Incheol, Daejeon 34141 (KR); YOON, Kuk-Jin, Daejeon 34141 (KR); CHO, Minkyu, Daejeon 34141 (KR)
(74) Representative: Bassil, Nicholas Charles
(86) International application number: PCT/KR2021/017852
(87) International publication number: WO 2022/119272

(57) **Abstract**

The present invention relates to a gas species identification method of a photoactive gas sensor using variable light irradiation. An object of the present invention is to provide a gas species identification method of a photoactive gas sensor using variable light irradiation capable of smoothly identifying a gas species without a selectivity difficult problem even with only a single chemical resistance-type gas sensor by irradiating light while periodically changing an amount of light at the time of irradiating the light to the photoactive gas sensor.

## Description

### [Technical Field]

The present invention relates to a gas species identification method of a photoactive gas sensor using variable light irradiation, and more particularly, to a method capable of identifying various gas species with at least one photoactive gas sensor by changing a method of irradiating light to the photoactive gas sensor.

### [Background Art]

As air pollution currently becomes more serious, damage to the human body and the ecosystem has increased day by day, and an effort for more effectively monitoring an air environment has been continuously made. In particular, recently, research and development of mobile air monitoring devices capable of measuring air pollution at various places rather than measuring air pollution only at a designated fixed place have been actively conducted. Due to characteristics of mobile devices driven by low-power power supplies such as batteries or solar cells, gas sensors installed in such mobile air monitoring devices should consume small power and have a small size. Meanwhile, in order to secure accuracy of monitoring data, it is preferable that sensitivity is high as compared with noise, and in order to collect a larger amount of data to improve the accuracy, the number of mobile devices themselves should be large, and thus, mass production of the mobile devices should be possible at a low price.

There are various types of gas sensors, such as a gas chromatography-based gas sensor, an optical gas sensor, an electrochemical gas sensor, and a chemical resistance-type gas sensor depending on a principle, a device configuration, or the like, that identifies gas species. Among them, the chemical resistance-type gas sensor is significantly fit for several conditions for the mobile devices, such as the low power, the miniaturization, the high sensitivity, the low cost, and the productivity as described above. The chemical resistance-type gas sensor uses a metal oxide semiconductor, silicon, a conductive polymer, graphene, a carbon nanotube, or the like, as a sensing material, and senses a target gas to be sensed using characteristics that electrical resistance of the sensing material changes when being exposed to the target gas. In order to show high sensitivity and a fast response by activating a chemical reaction in the chemical resistance-type gas sensor, the chemical resistance-type gas sensor is provided with a device that continuously supplies energy to the sensing material, for example, heats the sensing material to a high temperature or irradiates light to the sensing material. Korean Patent Laid-Open Publication No. 2018-0064112 (entitled "Semiconductor Device and Sensing Device" and published on 14 June, 2018) discloses an embodiment of a chemical resistance-type gas sensor including a semiconductor provided with a sensing material of which resistance is changed by light emitted from a light emitting part.

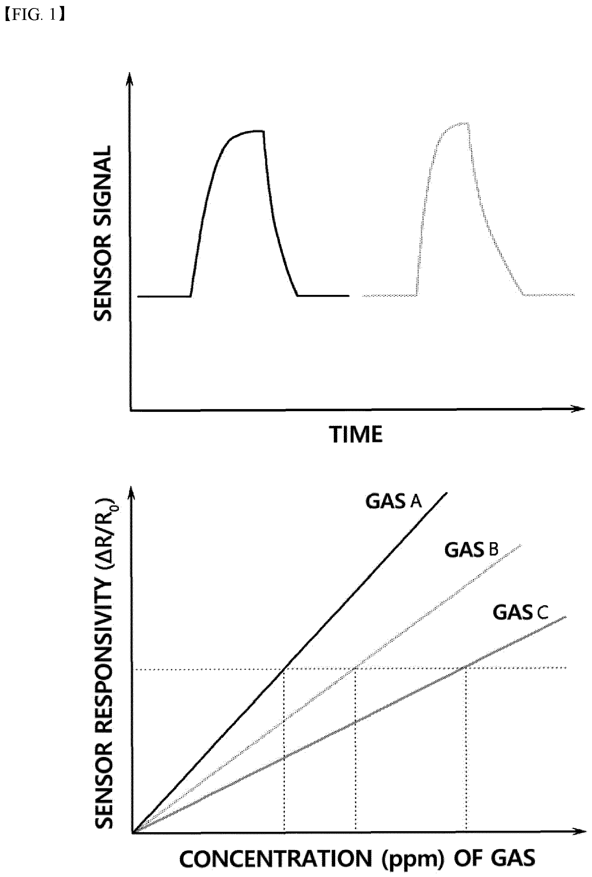

However, since the chemical resistance-type gas sensor responds to most reactive or toxic gases, there is a limitation that it is not possible to identify which gas has been sensed with only a single sensor signal, which is called a selectivity difficult problem. FIG. 1 illustrates a selectivity difficult problem of a chemical resistance-type gas sensor as an example. As illustrated in an upper view of FIG. 1, when the gas sensor is exposed to a gas, a sensor signal is generated due to a change in resistance of a sensing material of the gas sensor. However, a responsivity of the sensor signal is related not only to a gas species but also to a gas concentration, and as illustrated in a lower view of FIG. 1, when the gas species changes, a relationship between the gas concentration and the responsivity also changes. For example, when an experiment [a gas sensor is exposed to gas A] is performed in a fully controllable environment such as a laboratory, the sensor signal as illustrated in the upper view of FIG. 1 output as a result of injecting gas A may be confirmed and the responsivity may be obtained from the sensor signal, and the gas concentration may be obtained using a graph of gas A as illustrated in the lower view of FIG. 1. However, in a state in which it is not known which gas has been injected, even though the responsivity is obtained from the sensor signal, it may not be known whether to apply the responsivity to a graph of gas A graph or a graph of gas B, such it is impossible to determine the gas concentration.

A method most widely utilized in the related art in order to solve this selectivity problem is to simultaneously measure two or more species of gases using various types of sensor arrays with slightly different responsivities for each gas and identify the gas species from each other through signal processing. Korean Patent No. 1852074 (entitled "Electronic Nose System and Method for Gas Classification" and registered on 19 April, 2018) relates to a system using various types of sensor arrays that show different reactions depending on a gas species as such, and disclosure a technology of collecting sensor signal data using various types of polymer mixture sensors having interdigitated electrodes, micro-heating elements, and machine membranes included in the sensor arrays and reconstructing distortion data through a learning-based matrix operation to identify gas species. However, in such a manner of using various types of sensor arrays, there is a problem that as the number of sensors constituting the sensor array increases, total power consumption, a cost, a system size, and the like, increase in proportion to the number of sensors, such that there is a limitation in applying such a manner to a mobile gas sensor.

### [Patent Document]

### [Related Art Document]

1. Korean Patent Laid-Open Publication No. 2018-0064112 (entitled "Semiconductor Device and Sensing Device" and published on 14 June, 2018)
2. Korean Patent No. 1852074 (entitled "Electronic Nose System and Method for Gas Classification" and registered on 19 April, 2018)

### [Disclosure]

### [Technical Problem]

An object of the present invention is to provide a gas species identification method of a photoactive gas sensor using variable light irradiation capable of smoothly identifying a gas species without a selectivity difficult problem even with only a single chemical resistance-type gas sensor by irradiating light while periodically changing an amount of light at the time of irradiating the light to the photoactive gas sensor.

### [Technical Solution]

In one general aspect, a gas species identification method of a photoactive gas sensor including a sensing material of which a chemical reaction with a target gas is activated by light irradiation and electrical resistance is thus changed, an analysis unit acquiring and analyzing a response sensor signal generated by the sensing material, and a light irradiation unit irradiating light includes: a variable light irradiating step of irradiating light to the sensing material with a waveform of a predetermined light irradiation signal so that an amount of irradiated light varies over time by the light irradiation unit; a gas sensing step of activating a chemical reaction of the sensing material with the target gas by variable light irradiation to generate the response sensor signal; and a gas species identifying step of identifying a gas species of the target gas on the basis of a pre-stored waveform sample database for a waveform of the sensor signal according to the gas species by the analysis unit.

In this case, in the variable light irradiating step, the light may be irradiated with at least one waveform selected among a square wave, a triangular wave, a sine wave, an increase-type square wave, a random number-type square wave, and a multi-frequency sine wave.

In addition, in the gas species identifying step, by using a principle that a chemical reaction rate on a surface of the sensing material is different depending on a gas species, and thus, a transient aspect of the sensor signal appears differently for each gas species as an amount of irradiated light is varied, variable light may be irradiated to a pre-selected sample gas with a waveform of a pre-selected light irradiation signal to collect waveform samples of a sensor signal for the sample gas, thereby configuring a waveform sample database, and the gas species of the target gas may be identified on the basis of the waveform sample database of the sensor signal according to the gas species.

In addition, in the gas species identifying step, the sensor signal measured as the function according to time may be converted into a frequency spectrum through Fourier transform, and the gas species of the target gas may be identified using the frequency spectrum.

In addition, in the gas species identifying step, the gas species of the target gas may be identified using at least one method selected among principal component analysis (PCA), a support vector machine (SVM), an artificial neural network (ANN), and a deep neural network (DNN).

### [Advantageous Effects]

According to the present invention, in irradiating light to a chemical resistance-type gas sensor that uses a photoactive manner of activating a gas reaction by irradiating the light, unlike driving a light source in a constant amount of irradiated light that is generally conducted, it is possible to smoothly identify a gas species without a selectivity difficult problem with only a single chemical resistance-type gas sensor through variable light irradiation which irradiates light while periodically changing an amount of light.

In particular, according to the present invention, is possible to smoothly identify a plurality of gas species in spite of using only a single chemical resistance-type gas sensor fit for several conditions for a mobile device, such as low power, miniaturization, high sensitivity, a low cost, and productivity, and thus, the present invention is very suitable for being applied to a mobile air monitoring device capable of measuring air pollution at various places. In addition, according to the present invention, an existing sensor device may be utilized by changing only a light irradiation method without specially changing a configuration of the existing sensor device, and usability and compatibility are very excellent.

### [Description of Drawings]

FIG. 1 is views illustrating a selectivity difficult problem of a chemical resistance-type gas sensor.
FIG. 2 is views illustrating various variable light irradiation forms according to the present invention.
FIG. 3 is views illustrating signal processing processes of a gas sensor by a variable light irradiation method according to the present invention.
FIGS. 4A to 4C are views illustrating division of ethanol and hydrogen sulfide by the variable light irradiation method according to the present invention.
FIGS. 5A and 5B are views illustrating frequency spectrum classification of different gas species using a deep neural network according to the present invention.

### [Best Mode]

Hereinafter, a gas species identification method using variable light irradiation according to the present invention having the configuration as described above will be described in detail with reference to the accompanying drawings.

### [1] Gas species identification method of photoactive gas sensor using variable light irradiation according to the present invention

In the present invention, a photoactive gas sensor of chemical resistance-type gas sensors is used. The photoactive gas sensor is a sensor sensing a target gas using a change in a sensor signal due to a change in resistance in accordance with activation of a chemical reaction with the target gas by light irradiation. More specifically, the photoactive gas sensor basically includes a sensing material, an analysis unit, and a light irradiation unit.

The sensing material refers to a material of which a chemical reaction with the target gas is activated by light irradiation and electrical resistance is thus changed, and is a well-known material widely used in the photoactive gas sensor, and thus, is not particularly limited in the present specification.

The analysis unit serves to acquire and analyze a response sensor signal generated by the sensing material. When the sensing material is exposed to the target gas, a chemical reaction occurs between the sensing material and the target gas. In particular, such a chemical reaction is further activated by light irradiation. The sensor signal is electrical resistance of the sensing material, and as the sensing material chemically changes as such, the electrical resistance, that is, the sensor signal also changes. That is, it may be known whether or not the target gas has been sensed according to whether or the sensor signal has changed, and a concentration or the like of the target gas may also be known using how much the sensor signal has changed.

The light irradiation unit may be any device as long as it may irradiate light, but since the light irradiation unit should be able to appropriately irradiate light of a desired wavelength, a light emitting diode (LED), a lamp, an electroluminescence element, or the like, having a specific emission wavelength between 250 and 450 nm is generally used as the light irradiation unit. In the gas sensor, the sensing material and the analysis unit should be necessarily configured as an integrated device, but the light irradiation unit may be formed integrally with an assembly of the sensing material and the analysis unit (internal light source manner) or is not necessarily formed integrally with the assembly of the sensing material and the analysis unit and may be formed as a separate device from the assembly of the sensing material and the analysis unit (external light source manner).

Since it is well known that such a photoactive gas sensor has already been actively studied through various related art documents, a description for a material used as the sensing material, a circuit configuration of the analysis unit, a structure of the light irradiation unit, and the like, will be omitted herein.

Such a photoactive gas sensor is very suitable for being utilized in a mobile air monitoring device because it is significantly fit for several conditions for a mobile device, such as low power, miniaturization, high sensitivity, a low cost, and productivity, but responds to all of various species of gases as described above, and thus, there is a limitation that there is a selectivity difficult problem that it is not possible to identify which gas has been sensed with only a single sensor signal.

In the present invention, the photoactive sensor is driven using variable light irradiation which irradiates light while periodically changing an amount of light, unlike driving a light source in a constant amount of irradiated light that is generally conducted. As described above, a chemical reaction of the sensing material with the target gas is activated by the light irradiation, and accordingly, electrical resistance is changed, such that a sensor signal is generated. In this case, a chemical reaction rate on a surface of the sensing material is different depending on a gas species, and thus, a transient aspect of the sensor signal appears differently for each gas species as an amount of irradiated light is varied. The present invention may identify a gas species even with only a single sensor signal using this principle.

Each step of a gas species identification method of a photoactive gas sensor according to the present invention will be described. The gas species identification method of a photoactive gas sensor according to the present invention may include a variable light irradiating step, a gas sensing step, and a gas species identifying step. Each step will be described in detail below.

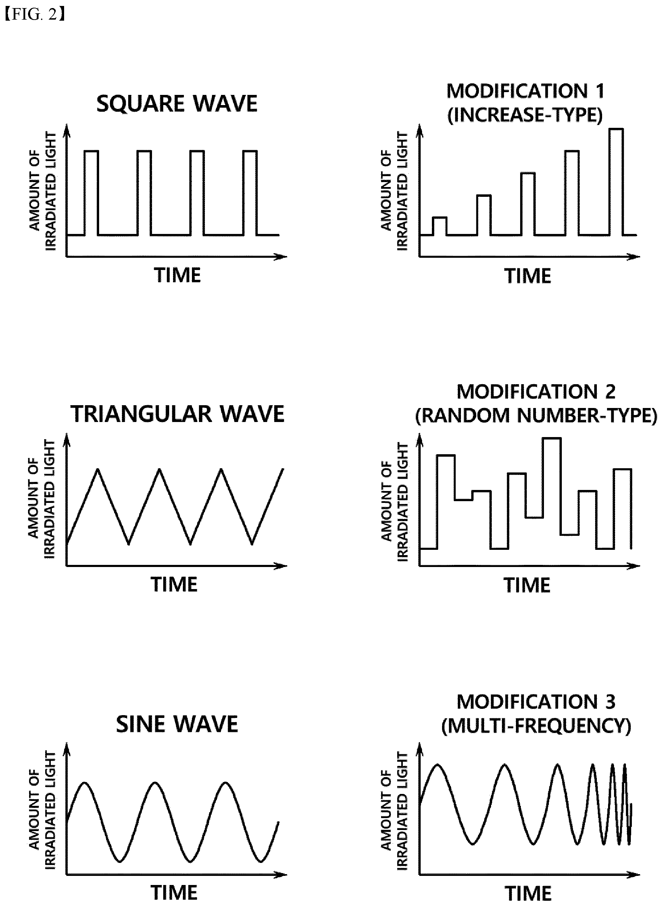

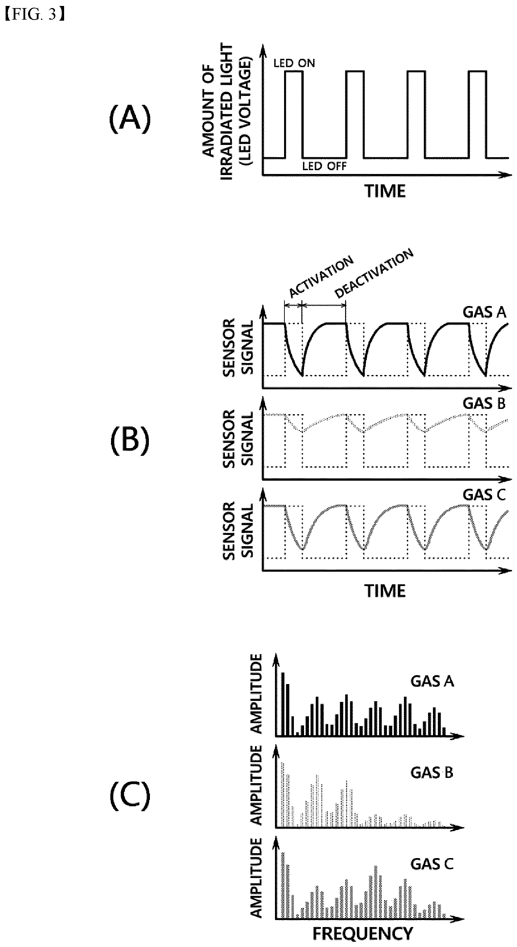

In the variable light irradiating step, the light irradiation unit irradiates light to the sensing material with a waveform of a predetermined light irradiation signal so that an amount of irradiated light varies over time. As described above, the photoactive gas sensor according to the related art has used a manner of maintaining a constant amount of irradiated light, but in the present invention, an amount of irradiated light variously varies with time. FIG. 2 illustrates several examples of various variable light irradiation forms according to the present invention, and illustrates a square wave, a triangular wave, a sine wave, an increase-type square wave, a random number-type square wave, and a multi-frequency sine wave. A waveform having any form may be used as a waveform of a light irradiation signal as long as it is a waveform that enables a transient aspect of a sensor signal according to a gas species to be recognized better, and the present invention is not limited to FIG. 2. FIG. 3 illustrates signal processing processes of a gas sensor by a variable light irradiation method according to the present invention, and FIG. 3A illustrates an example in a case where the light irradiation signal has a square waveform.

In the gas sensing step, a chemical reaction of the sensing material with the target gas is activated by variable light irradiation to generate a response sensor signal. As described above, the sensor signal may be obtained as an electrical resistance value of the sensing material, and when an amount of irradiated light increases, the sensor signal decreases due to generation of a photocurrent and when the amount of irradiated light decreases, the sensor signal increases due to a decrease in the photocurrent. In this case, a degree and a speed of the sensor signal increase/decrease in a specific gas atmosphere are changed. FIG. 3B illustrates an example in which all of waveforms of gas A, gas B, and gas C appear differently even through variable light irradiation according to the light irradiation signal as illustrated in FIG. 3A is performed. That is, since the chemical reaction rate on the surface of the sensing material is different depending on the gas species, the transient aspect of the sensor signal appears differently for each gas species as the amount of irradiated light is varied, and as described above, in the present invention, the gas species of the target gas is identified using this principle.

In the gas species identifying step, the analysis unit identifies the gas species of the target gas on the basis of a pre-stored waveform sample database for a waveform of the sensor signal according to the gas species. A waveform sample of a sensor signal for a pre-selected sample gas may be obtained by irradiating variable light to the pre-selected sample gas with a waveform of a pre-selected light irradiation signal using the principle that the transient aspect of the sensor signal appears differently for each gas species as described above, and it may be identified which gas a newly sensed unconfirmed target gas is by comparing a waveform of a sensor signal of the unconfirmed target gas with the wavelength sample described above.

A more detailed description will be provided with reference to FIG. 3. For example, in a laboratory where all conditions are known and all environments may be controlled, when variable light irradiation is made according to a light irradiation signal having a square waveform as illustrated in FIG. 3A, waveforms of different sensor signals appear for each of gas A, gas B, and gas C, as illustrated in FIG. 3B. In this case, it may be known that a form of the light irradiation signal is a "square wave" and which waveforms of sensor signals of "gas A", "gas B", and "gas C" appear, and waveform samples of these sensor signals may be collected for various desired gases to configure a waveform sample database. After experiments are performed on various gases with the light irradiation signal as desired to configure a sample database of sensor signals according to the gas species as described above, when an unknown target gas is sensed in an actual field, a gas species of the target gas may be identified on the basis of this database.

In this case, in a case where comparative analysis is performed using the sensor signal measured as a function according to time as it is, there is a risk that it will take a longer time to perform the comparison and accuracy will decrease. In order to solve such a problem, in the gas species identifying step, it is preferable to convert the sensor signal measured as the function according to time into a frequency spectrum through Fourier transform and identify the gas species of the target gas using the frequency spectrum. FIG. 3C illustrates that sensor signals measured as a function according to time of each of gas A, gas B, and gas C illustrated in FIG. 3B are converted into frequency spectra through Fourier transform. When the sensor signals are converted into the frequency spectra as described above, comparative analysis may become easier, and accordingly, an identification speed and accuracy may be further improved.

### [2] Example of gas species identification method according to the present invention

Hereinafter, an experimental example of identifying a gas species using the gas species identification method of a photoactive gas sensor using variable light irradiation of the present invention will be introduced.

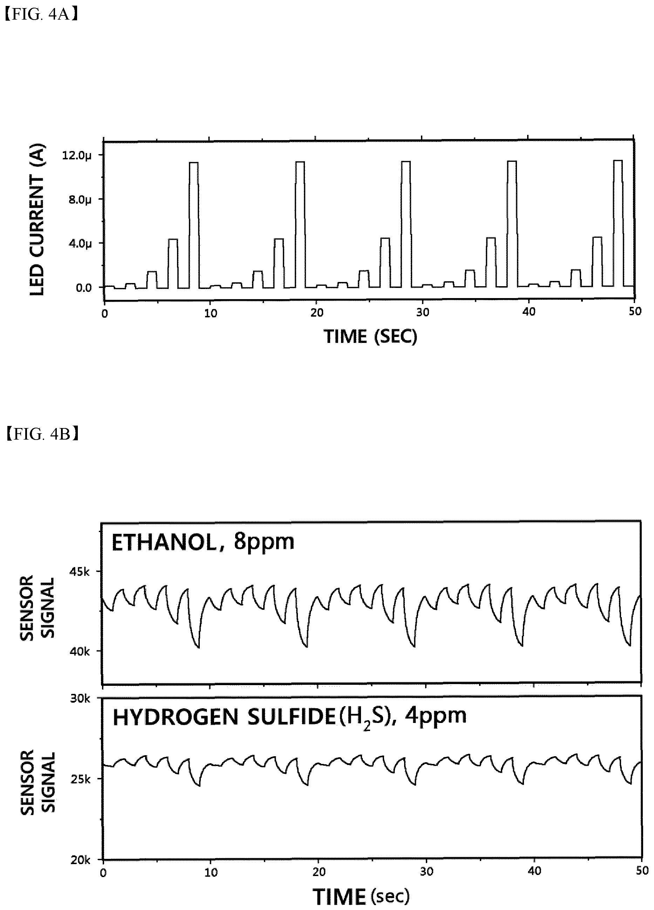

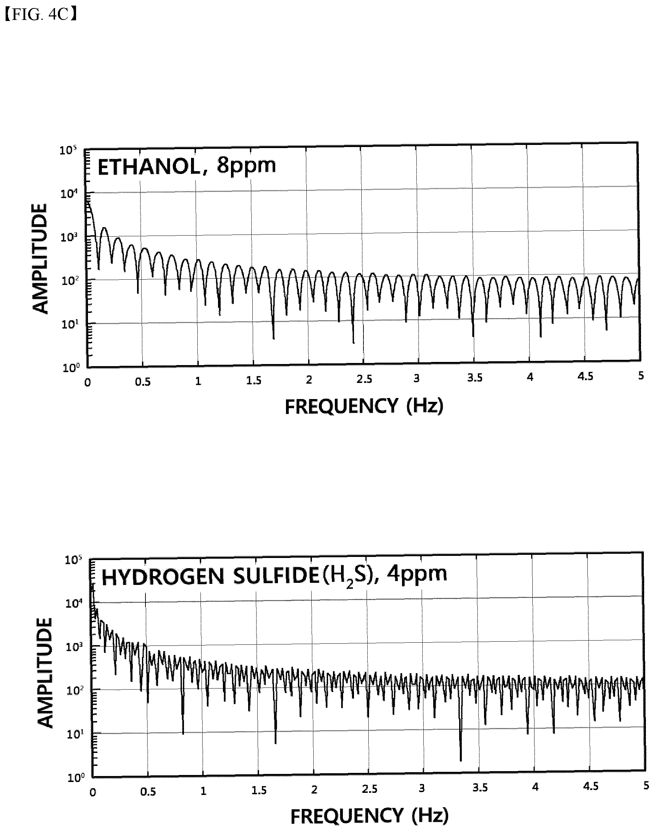

FIGS. 4A to 4C are views of experimental examples illustrating division of ethanol and hydrogen sulfide by the variable light irradiation method according to the present invention. This is a process of configuring a waveform sample database of sensor signals. First, a light irradiation signal having an increase-type square waveform was applied to a photoactive gas sensor disposed in an atmosphere of ethanol (8 ppm) and hydrogen sulfide (4 ppm) at a period of 10 seconds as illustrated in FIG. 4A. In this case, sensor signals appeared in response to the light irradiation signal of FIG. 4A, and sensor signal graphs for each of ethanol and hydrogen sulfide were obtained as illustrated in FIG. 4B. FIG. 4C is graphs obtained by transforming the sensor signals having a form of a function according to time illustrated in FIG. 4B into frequency spectra through Fourier transform. It may be intuitively seen from FIGS. 4B and 4C that forms of the sensor signals and the frequency spectra in each of ethanol and hydrogen sulfide are very different from each other. Only examples of ethanol and hydrogen sulfide have been illustrated in FIG. 4, but sensor signal samples may be made for various gas species as desired in such a manner to configure a database.

When the database is configured in such a manner, it may be now identified which gas an unconfirmed target gas is (that is, a gas species of the unconfirmed target gas) by obtaining a sensor signal and a frequency spectrum for the unconfirmed target gas and then comparing and analyzing the sensor signal and the frequency spectrum with a sample of the database. In this case, at the time of comparing and analyzing the sensor signal and frequency spectrum of the unconfirmed target gas with the sample of the database, various machine learning techniques such as principal component analysis (PCA), a support vector machine (SVM), an artificial neural network (ANN), and a deep neural network (DNN) may be used.

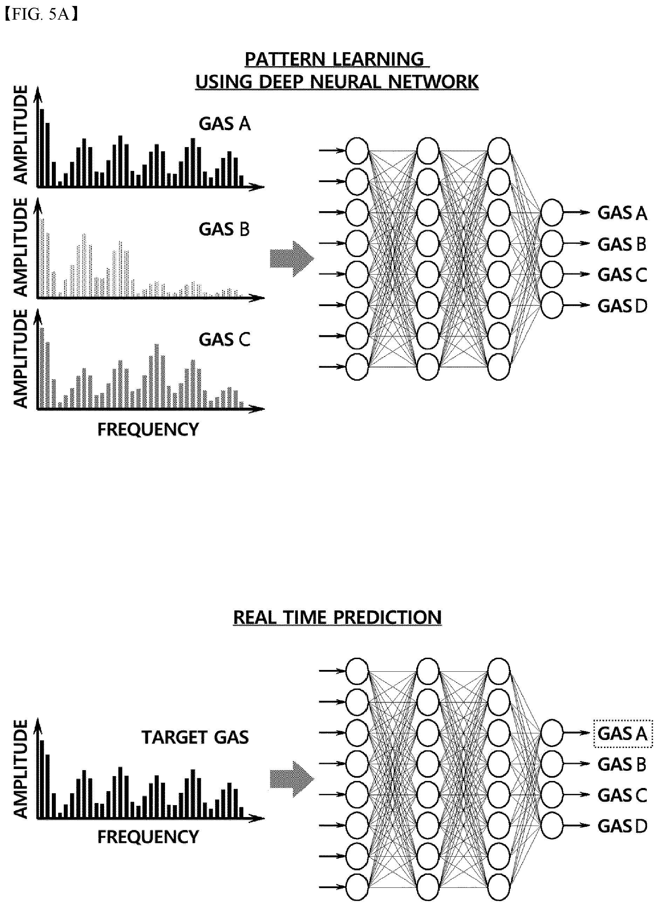

An upper view of FIG. 5A illustrates a pattern learning process using such database construction and machine learning technique, and in the upper view of FIG. 5A, a deep neural network is used as an example. As illustrated in the upper view of FIG. 5A, frequency spectra of sensor signals for each of various gases such as gas A, gas B, and gas C may be collected to configure a database, after a pattern is learned using a deep neural network, a gas species of the unconfirmed target gas may be now identified.

A lower view of FIG. 5A illustrates a process of predicting and identifying a gas species in real time by comparing and analyzing the unconfirmed target gas in a state in which the database is constructed and the pattern is learned as described above. As illustrated in the lower view of FIG. 5A, when a frequency spectrum of the sensor signal is input to a system in a state in which it is not known which gas the target gas is, it may be identified which gas the unconfirmed target gas is through machine learning pattern recognition. In the lower view of FIG. 5A, a frequency spectrum of "gas A" was used as a frequency spectrum of a "target gas" in an unconfirmed state as it is in the upper view of FIG. 5A for easy understanding, and it is illustrated in the lower view of FIG. 5A that it is identified that the frequency spectrum of the "target gas" in the unconfirmed state is the frequency spectrum of "gas A" through pattern recognition.

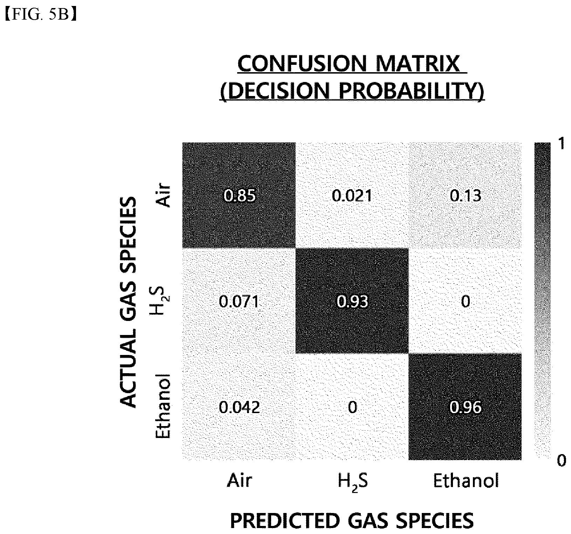

FIG. 5B illustrates an example in which identification of three species of gases, that is, ethanol, hydrogen sulfide, and air, is performed using the database constructed through the process of FIGS. 4A to 4C. In detail, FIG. 5B illustrates a confusion matrix between a predicted gas species predicted through real-time prediction and an actual gas species actually used as an unconfirmed target gas. As illustrated in FIG. 5B, it may be confirmed that the gas species identification method according to the present invention is used, such that even though only a single chemical resistance-type (photoactive) gas sensor is used, decision accuracy of ethanol is 96%, decision accuracy of hydrogen sulfide is 93%, decision accuracy of air is 85%, and thus, average decision accuracy is 91%, which is very excellent.

As described above, the gas species identification method of a photoactive gas sensor using variable light irradiation according to the present invention may identify the gas species smoothly without a selectivity difficult problem only with only one photoactive gas sensor. In a case of actually implementing a sensor device, when the sensor device regards low power and miniaturization as a mobile monitoring device as important, a gas species identification function may be excellently implemented even though the sensor device includes only a single gas sensor, by applying the gas species identification method of a photoactive gas sensor using variable light irradiation according to the present invention. Meanwhile, a method of identifying a gas species using only a single gas sensor has been consistently described above, but when the present invention is to be applied at the time of actually implementing the sensor device, the sensor device does not need to be necessarily limited to including only a single gas sensor. That is, the sensor device may also include a plurality of gas sensors, and in this case, it may be expected that more various gas species may be more accurately identified by collecting and further analyzing gas species identification results of each of the plurality of gas sensors.

The present invention is not limited to the embodiments described above, and may be applied to various fields. In addition, the present invention may be variously modified by those skilled in the art to which the present invention pertains without departing from the gist of the present invention claimed in the claims.

### [Industrial Applicability]

According to the present invention, it is possible to smoothly identify a gas species without a selectivity difficult problem with only a single chemical resistance-type gas sensor through variable light irradiation that irradiates light while periodically changing an amount of light. Therefore, the present invention is advantageous in miniaturization and cost reduction of a device, and is particularly suitable for being applied to a mobile air monitoring device.

## Claims

1. A gas species identification method of a photoactive gas sensor including a sensing material of which a chemical reaction with a target gas is activated by light irradiation and electrical resistance is thus changed, an analysis unit acquiring and analyzing a response sensor signal generated by the sensing material, and a light irradiation unit irradiating light, comprising:
a variable light irradiating step of irradiating light to the sensing material with a waveform of a predetermined light irradiation signal so that an amount of irradiated light varies over time by the light irradiation unit;
a gas sensing step of activating a chemical reaction of the sensing material with the target gas by variable light irradiation to generate the response sensor signal; and
a gas species identifying step of identifying a gas species of the target gas on the basis of a pre-stored waveform sample database for a waveform of the sensor signal according to the gas species by the analysis unit.

2. The gas species identification method of a photoactive gas sensor of claim 1, wherein in the variable light irradiating step,
the light is irradiated with at least one waveform selected among a square wave, a triangular wave, a sine wave, an increase-type square wave, a random number-type square wave, and a multi-frequency sine wave.

3. The gas species identification method of a photoactive gas sensor of claim 1, wherein in the gas species identifying step,
by using a principle that a chemical reaction rate on a surface of the sensing material is different depending on a gas species, and thus, a transient aspect of the sensor signal appears differently for each gas species as an amount of irradiated light is varied,
variable light is irradiated to a pre-selected sample gas with a waveform of a pre-selected light irradiation signal to collect waveform samples of a sensor signal for the sample gas, thereby configuring a waveform sample database, and
the gas species of the target gas is identified on the basis of the waveform sample database of the sensor signal according to the gas species.

4. The gas species identification method of a photoactive gas sensor of claim 1, wherein in the gas species identifying step,
the sensor signal measured as the function according to time is converted into a frequency spectrum through Fourier transform, and
the gas species of the target gas is identified using the frequency spectrum.

5. The gas species identification method of a photoactive gas sensor of claim 1, wherein in the gas species identifying step,
the gas species of the target gas is identified using at least one method selected among principal component analysis (PCA), a support vector machine (SVM), an artificial neural network (ANN), and a deep neural network (DNN).
